# EUROPÄISCHE PATENTSCHRIFT

(11) **EP 0 569 869 B1**
(45) Veröffentlichungstag und Bekanntmachung des Hinweises auf die Patenterteilung: **13.01.1999**
(21) Anmeldenummer: 93107417.3
(22) Anmeldetag: 07.05.1993
(51) Int. Cl.: C07H 15/04, A61K 7/08, A61K 7/50, C11D 1/52, C11D 1/56

(54) **Lactobionsäureamidzusammensetzungen und deren Verwendung**
Lactobionic acid amide compositions and their use
Composés d'amides de l'acide lactobionique et leur utilisation

(30) Priorität: 11.05.1992 DE 4215478
(43) Veröffentlichungstag der Anmeldung: 18.11.1993
(73) Patentinhaber: Solvay Deutschland GmbH, D-30173 Hannover (DE)
(72) Erfinder: Gerling, Klaus-Günter, W-3014 Laatzen 5 (DE); Wendler, Kornelia, W-3163 Sehnde 8 (DE); Joisten, Sabine, W-3012 Langenhagen (DE); Schreer, Claudia, W-3209 Schellerten (DE)
(74) Vertreter: Lauer, Dieter, Dr.

(56) Entgegenhaltungen:
- EP-A- 550 278
- EP-A- 550 281
- EP-A- 551 675
- DE-A- 1 155 771
- FR-A- 2 523 962
- US-A- 2 752 334
- CARBOHYDRATE RESEARCH. Bd. 67, 1978, AMSTERDAM NL Seiten C1 - C3 WILLIAMS T.J. ET AL 'Synthesis of a new class of model glycolipids'
- ARCHIVES OF BIOCHEMISTRY AND BIOPHYSICS Bd. 195, Nr. 1, Juni 1979, NEW YORK (US) Seiten 145 - 151 WILLIAMS T.J. ET AL 'A New Class of Model Glycolipids: Synthesis, Characterization, and Interaction with Lectins'

## Beschreibung

Die Erfindung betrifft neue Lactobionsäureamidzusammensetzungen, welche aus Lactobionsäure und Fettamingemischen erhaltene Amidgemische darstellen, sowie deren Verwendung.

In dem deutschen Patent Nr. 11 55 771 wird die Herstellung von Maltobionsäure-, Lactobionsäure- oder Cellobionsäure-N-alkylamiden mit 6 bis 12 Kohlenstoffatomen im Alkylrest durch Umsetzung eines entsprechenden Säurelactons mit dem Alkylamin in Dimethylformamid beschrieben und angegeben, daß diese Amide grenzflächenaktive Eigenschaften besitzen.

Aus US 2,752,334 sind N-substituierte Lactobionamide bekannt, die emulgierende und antimykotische Eigenschaften aufweisen und somit z. B. für die Käseherstellung geeignet sind.

Williams et al. (Arch. Biochem. Biophys. Vol 195, 1979, S. 145 - 151) nennt Lactobionsäure-N-alkylamide mit 14 oder 16 Kohlenstoffatomen im Alkylrest als Modellsubstanzen für Glykolipide.

Die älteren Rechte EP 551 675, EP 550 278 und EP 550 281 offenbaren die Verwendung von Lactobionsäureamiden in Reinigungs- oder Körperpflegemitteln.

Allen Veröffentlichungen ist gemeinsam, daß die Umsetzung der Lactone mit einzelnen Fettaminen erfolgt, es werden keine Fettamingemische natürlicher Herkunft verwendet.

Der vorliegenden Erfindung liegt die Aufgabe zugrunde, neue grenzflächenaktive, aus nachwachsenden Rohstoffen erhältliche und biologisch abbaubare Zusammensetzungen bereitzustellen, welche als Bestandteile mit detergierenden, schaumstabilisierenden und/oder verdickend wirksamen Eigenschaften für flüssige, in wäßrigen Systemen einsetzbare Wasch- oder Spülmittelzusammensetzungen und/oder Kosmetika eingesetzt werden können.

Es wurde nun gefunden, daß Amidgemische aus Amiden der Lactobionsäure mit höheren Fettamingemischen neben guten grenzflächenaktiven Eigenschaften auch eine gute Löslichkeit und gute schaumstabilisierende Eigenschaften in wäßrigen Systemen, aufweisen und sich aufgrund ihrer hervorragenden anwendungstechnischen Eigenschaften als weichmachende, detergierende, emulgierende, schaumstabilisierende und/oder verdickend wirksame Bestandteile von Wasch-, Spül- und Reinigungsmittelzusammensetzungen, Weichspülmitteln, insbesondere Textilweichspülmitteln, und kosmetische Formulierungen eignen.

Gegenstand der Erfindung sind daher Lactobionsäureamidzusammensetzungen aus Amiden der Lactobionsäure mit einem Gemisch primärer Fettamine, welches mindestens 30 Gew.-% an Fettaminen mit einer Kettenlänge von 14 bis 18 Kohlenstoffatomen enthält, und deren Verwendung.

Als "primäre Fettamine" werden im Rahmen der vorliegenden Erfindung primäre Amine bezeichnet, welche einen dem aliphatischen Rest einer Fettsäure entsprechenden geradkettigen aliphatischen Rest enthalten. Fettamine können z.B. technisch aus Fettsäuren erhalten werden, indem diese zunächst in ihre Nitrile überführt werden, welche anschließend zu Aminen reduziert werden.

Die erfindungsgemäßen Lactobionsäureamidzusammensetzungen können durch Umsetzen von Lactobionsäure oder reaktiven Lactobionsäurederivaten, insbesondere Lactobionsäurelacton, mit einem entsprechenden Fettamingemisch erhalten werden. Zur Herstellung der erfindungsgemäßen Lactobionsäureamidzusammensetzungen werden Fettamingemische eingesetzt, die mindestens 30 Gew.-% an Fettaminen mit einer Kettenlänge von 14 bis 18 Kohlenstoffatomen enthalten. Als besonders günstig erweisen sich Fettamingemische mit einem Anteil von mindestens 90 Gew.-%, beispielsweise 90 bis 98 Gew.-% an Fettaminen mit einer Kettenlänge von 16 bis 18 Kohlenstoffatomen. Vorzugsweise werden Fettamingemische mit einem Anteil von ca. 5 bis 85 Gew.-%, insbesondere 35 bis 85 Gew.-% an einfach ungesättigten Fettaminen eingesetzt.

Lactobionsäure(= 4-(β-D-Galacto)-D-gluconsäure) und Lactobionsäurelacton sowie deren Herstellung sind bereits bekannt. Lactobionsäure kann beispielsweise auf bekannte Weise durch Oxidation von Lactose erhalten werden.

Bevorzugt werden Fettamingemische eingesetzt, welche aus natürlich vorkommenden Fettsäuregemischen gewonnen werden. Der Anteil von einfach ungesättigten Fettaminen schwankt in diesen Fettamingemischen zwischen ca. 5 und 85 Gew.-%. Beispiele solcher Fettamingemische sind Kokosfettamin aus dem aus Kokosfett stammenden Fettsäuregemisch, Talgamin und hydriertes Talgamin aus dem aus Talg stammenden Fettsäuregemisch, Oleylamin aus dem aus Sonnenblumenöl und/oder Sojaöl stammenden Fettsäuregemisch. Kokosfettamin enthält z.B. ca. 50 Gew.-% an gesättigten C₁₂-Fettaminen, ca. 18 Gew.-% an gesättigten C₁₄-Fettaminen sowie ca. einen Anteil von ca. 7 Gew.-% an ungesättigten C₁₈-Fettaminen. Hydriertes Talgamin enthält z.B. ca. 30 Gew.-% an gesättigten C₁₆-Fettaminen, ca. 60 Gew.-% an gesättigten C₁₈-Fettaminen sowie noch einen Anteil von ca. 3 Gew.-% an ungesättigten Fettaminen mit 14 bis 18 Kohlenstoffatomen. Talgamin enthält z.B. ca. 29 Gew.-% an gesättigten C₁₆-Fettaminen, ca. 23 Gew.-% an gesättigten C₁₈-Fettaminen sowie einen Anteil von ca. 42 Gew.-% an ungesättigten Fettaminen mit 14 bis 18 Kohlenstoffatomen. Aus Sonnenblumenöl gewonnenes Oleylamin enthält z.B. ca. 14 Gew.-% an gesättigten Fettaminen mit 12 bis 18 Kohlenstoffatomen und ca. 85 Gew.-% an ungesättigten Fettaminen mit 14 bis 18 Kohlenstoffatomen. Aus Sojaöl gewonnenes Oleylamin (= Sojaamin) enthält z.B. ca. 16 Gew.-% an gesättigten C₁₆-Fettaminen, ca. 15 Gew.-% an gesättigten C₁₈-Fettaminen sowie einen Anteil von ca. 63 Gew.-% an ungesättigten Fettaminen mit 14 bis 18 Kohlenstoffatomen.

Weiterhin umfaßt die Erfindung ein Verfahren zur Herstellung der erfindungsgemäßen Lactobionsäureamidzusammensetzungen. Die Herstellung der erfindungsgemäßen Lactobionsäureamidzusammensetzungen erfolgt durch die Umsetzung von Lactobionsäure oder deren reaktionsfähigen Derivaten, insbesondere Lactobionsäurelacton, mit dem Fettamingemisch zu der erfindungsgemäßen Lactobionsäureamidzusammensetzung in einem niederen Alkylalkohol, vorzugsweise in einem niederen Alkylalkohol mit 1 bis 4 Kohlenstoffatomen, insbesondere Methanol, Isopropanol oder Ethanol. Dabei wird die Umsetzung in der Regel so durchgeführt, daß man die Lactobionsäure, oder vorzugsweise das Lactobionsäurelacton, in dem Alkohol löst bzw. suspendiert und das Fettamin-Gemisch langsam unter Rühren zutropft. Anschließend wird noch für ca. 1 bis 3 Stunden bei 0 bis 5 °C gerührt bzw. homogenisiert. Zur vollständigen Auskristallisation des Reaktionsproduktes wird die Lösung bzw. Suspension dann noch gegebenenfalls für 1 bis 18 Stunden stehengelassen. Die erfindungsgemäße Lactobionsäureamidzusammensetzung fällt als Niederschlag aus, der gewaschen und im Trockenschrank gegebenenfalls unter Vakuum getrocknet wird.

Es ist überraschend, daß die Herstellung der erfindungsgemäßen Lactobionsäureamidzusammensetzungen auch in einer Suspension, wie sie bei der Umsetzung in Ethanol oder Isopropanol vorliegt, gelingt und so die Verwendung von bisher vorgeschlagenem Dimethylformamid, welches toxikologisch bedenklich ist, vermieden werden kann.

Ein Vorteil der erfindungsgemäßen Amidgemische liegt darin, daß sie ausschließlich aus Komponenten natürlichen Ursprungs wie Lactose (im Lactobionsäurerest) und Fettsäure (im langkettigen Amidrest) bestehen. Ferner enthalten sie aufgrund ihrer Herstellung in einem niederen Alkohol wie Isopropanol oder Ethanol keine Rückstände von toxikologisch problematischen Substanzen. Damit sind sie hautfreundlich und gut biologisch abbaubar.

Die erfindungsgemäßen Lactobionsäureamidzusammensetzungen können auf Grundlage einer gesicherten Rohstoffbasis leicht gewonnen werden und sind somit auch in wirtschaftlicher Hinsicht vorteilhaft.

Die erfindungsgemäßen Lactobionsäureamidzusammensetzungen zeigen überraschend gute anwendungstechnische Eigenschaften. In wässrigen Systemen besitzen die erfindungsgemäßen Lactobionsäureamidzusammensetzungen gute grenzflächenaktive Eigenschaften zusammen mit einem günstigen Schaumverhalten. So zeigen die erfindungsgemäßen Lactobionsäureamidzusammensetzungen nicht nur ein stärkeres Schaumvermögen, sondern auch eine um ein Vielfaches niedrigere und damit günstigere kritische Micellenbildungskonzentration als z.B. das an sich bekannte Lactobionsäure-N-dodecylamid als Reinsubstanz. Die erfindungsgemäßen Zusammensetzungen sind somit gut als detergierende (sowohl reinigend als auch weichmachend), emulgierende, schaumstabilisierend und/oder verdickend wirksame grenzflächenaktive Bestandteile in Wasch-, Spül- und Reinigungsmittelzubereitungen, u.a. auch Weichspülern, und kosmetischen Formulierungen verwendbar. Je nach Anwendungszweck können dabei in diesen Zubereitungen selbstverständlich noch weitere an sich gebräuchliche Bestandteile enthalten sein, so daß die Konzentration der erfindungsgemäßen Lactobionsäureamidzusammensetzungen in diesen Zubereitungen in weiten Bereichen, z.B. zwischen 0,1 bis 80 Gew.-%, bezogen auf die Gesamtzubereitung, varieren kann.

Mit guter Detergenz-Wirkung können die erfindungsgemäßen Lactobionsäureamidzusammensetzungen z.B. als Tensidbestandteil in Wasch-, Reinigungs- und Spülmitteln eingesetzt werden. Vorteilhaft werden die erfindungsgemäßen Lactobionsäureamidzusammensetzungen dabei in einer Konzentration von 0,1 bis 50 Gew.-% verwendet. Insbesondere Lactobionsäure-N-kokosamid zeichnet sich durch die Bildung eines stabilen Schaums neben einer überraschend hohen Löslichkeit aus, was z.B. bei der Verwendung in Textilwasch- und Reinigungsmitteln, Geschirrhandspülmitteln oder kosmetischen Formulierungen wie z.B. Schaumbädern oder Haarshampoos vorteilhaft ist.

Die erfindungsgemäßen Amidgemische sind auch gut geeignet als emulgierend und/oder verdickend wirksame Bestandteile von kosmetischen Formulierungen. Als emulgierende Zusätze eignen sich beispielsweise Lactobionsäure-N-oleylamid oder Lactobionsäure-N-talgamid z.B. in Konzentrationen von 0,5 bis 15 Gew.-%, insbesondere 5 bis 15 Gew.-%, bezogen auf die Gesamtelmulsion. Als Verdickungsmittel ist insbesondere Lactobionsäure-N-oleylamid gut geeignet, das aufgrund seiner guten Wasserlöslichkeit in zur Ausbildung einer vernetzenden Struktur ausreichenden Konzentration angewendet werden kann. Vorteilhaft sollte bei der Verwendung von Lactobionsäure-N-oleylamid als Verdickungsmittel dessen Konzentration zwischen 2 bis 15 Gew.-%, bezogen auf die Gesamtbereitung, betragen. Je nach eingesetzter Konzentration können dabei dickflüssige, zähflüssige nicht fließfähige halbfeste oder halbfeste fast schnittfeste Gele hergestellt werden. Eine wäßrige Lösung mit 6 Gew.-% Lactobionsäure-N-oleylamid hat beispielsweise eine zähflüssige Konsistenz, eine wäßrige Lösung mit 12 Gew.-% Lactbionsäure-N-oleylamid hat die Konsistenz eines klaren fast schnittfesten Gels.

Die mit den erfindungsgemäßen Substanzen hergestellten Emulsionen weisen in ihrer Konsistenz eine sehr gute Langzeitstabilität auf.

Darüber hinaus sind die erfindungsgemäßen Lactobionsäureamidzusammensetzungen auch sehr gut als weichmachende Wirkstoffe in Weichspülmitteln, insbesondere Textilweichspülmitteln, verwendbar. Hierzu eignen sich insbesondere Lactobionsäure-N-oleylamid oder Lactobionsäure-N-talgamid. Mit einer wäßrigen Lösung, die 0,01 bis 15 Gew.-%, insbesondere 0,01 bis 5 Gew.-% eines erfindungsgemäßen Lactobionsäure-N-amidgemisch enthält, wird eine gute textilweichmachende Wirkung erreicht. Neben den in Weichspülmitteln üblichen Hilfstoffen können dabei gegebenenfalls auch noch andere weichmachende Substanzen enthalten sein. So können in den Formulierungen als Textilweichspülmittel neben der erfindungsgemäßen Lactobionsäureamidzusammensetzung noch andere, üblicherweise in Textilweichspülmitteln enthaltene Bestandteile, wie z.B. Duftstoffe, Farbstoffe, Pigmente, Trübungsmittel, optische Aufheller, korrosionshemmende Mittel, wasserlösliche Polymere oder antistatische Mittel in den für diese Bestandteile üblichen Mengen enthalten sein. Zweckmäßigerweise werden die erfindungsgemäßen Lactobionsäureamidzusammensetzungen in einer Konzentration von 1 bis 80 Gew.-%, bezogen auf die Gesamtformulierung des Textilweichspülmittels, eingesetzt. Es ist überraschend, daß die erfindungsgemäßen Lactobionsäureamidzusammensetzungen so gute weichmachende, insbesondere textilweichmachende, Eigenschaften aufweisen, obwohl sie sich in ihrer Struktur sehr stark von den bisherigen weichmachenden Wirkstoffen in Textilweichspülmitteln, die zumeist kationische, quarternäre Ammoniumverbindungen sind, unterscheiden. Gegenüber den bisher als textilweichmachende Wirkstoffverwendeten kationischen Ammoniumverbindungen, die bekanntermaßen biologisch schlecht abbaubar und dadurch zu einer stärkeren Umweltbelastung beitragen, unterscheiden sich die erfindungsgemäßen Lactobionsäureamidzusammensetzungen vorteilhaft durch ihre biologisch gut abbaubare Struktur.

Die nachfolgenden Beispiele sollen die Erfindung weiter erläutern, ohne sie jedoch in ihrem Umfang zu begrenzen.

### Beispiele

### 1. Herstellung von Lactobionsäure-N-oleylamid:

### a) Herstellung in Methanol:

500 g Lactobionsäurelacton wurden in einem Mörser feingerieben und dann portionsweise bei 50 bis 60 °C in 1,6 l Methanol gelöst. In diese Lösung wurden 324,4 g Oleylamin unter Rühren langsam zugetropft. Diese gesamte noch klare Lösung wurde 1 Stunde unter Eiskühlung gerührt und anschließend für ca. 12 Stunden stehengelassen. Es fiel ein weißer Niederschlag aus, der abfiltriert, mit Methanol gewaschen und dann im Vakuumtrockenschrank bei 40 bis 50 °C getrocknet wurde. Die Ausbeute betrug 93 Gew.-% Lactobionsäure-N-oleylamid, bezogen auf Lactobionsäurelacton.

### b) Herstellung in Isopropanol:

500 g Lactobionsäurelacton wurden in einem Mörser feingerieben und dann bei 50 bis 60 °C portionsweise mit Hilfe eines Homogenisators in 1000 ml Isopropanol suspendiert. Als Homogenisator wurde eine an sich bekannte Feinzerkleinerungsmühle (Typ PUC-NA 60) zur besseren Durchmischung der erhaltenen Suspension eingesetzt. 326 g Oleylamin wurden auf ca. 40 bis 50 °C erwärmt und tropfenweise unter weiterer Homogenisation der Suspension zugesetzt. Die gesamte Suspension wurde noch ca. 1 Stunde unter Eiskühlung homogenisiert und anschließend für ca. 12 Stunden stehengelassen. Ein weißer Niederschlag wurde abfiltriert, mit Isopropanol gewaschen und dann im Vakuumtrockenschrank bei 40 bis 50 °C getrocknet. Die Ausbeute betrug 90 Gew.-% Lactobionsäure-N-oleylamid, bezogen auf Lactobionsäurelacton.

In analoger Weise, wie für Isopropanol in Beispiel 1. b) beschrieben, wurde Lactobionsäure-N-oleylamid auch in Ethanol als Lösungsmittel hergestellt.

Wie beispielhaft für Lactobionsäure-N-oleylamid beschrieben, erfolgte auch die Herstellung der übrigen erfindungsgemäßen Lactobionsäureamidzusammensetzungen.

### 2. a) Bestimmung des Schaumvermögens:

Das Schaumvermögen der erfindungsgemäßen Lactobionsäureamidzusammensetzungen wurde nach dem modifizierten Ross-Miles-Verfahren in Anlehnung an DIN 53902, Teil 2, wie nachfolgend beschrieben, bestimmt.

Zur Bestimmung wurden 1.000 ml wäßrige Lösung mit 0,1 Gew.-% erfindungsgemäßen Lactobionsäureamidgemisch hergestellt.

In einer Apparatur, die aus zwei übereinander angeordneten thermostatierten Glasgefäßen besteht, wurden ca. 20 ml der zuvor auf 50 °C temperierten Lösung im unteren Gefäß vorgelegt und 500 ml der Lösung in das obere Gefäß gefüllt. Durch eine Kapillare ließ man die Lösung aus dem oberen Gefäß in die vorgelegte Lösung im unteren Gefäß einlaufen. Die Höhe des hierbei entstandenen Schaumes wurde nach 30 Sekunden, 3 Minuten und 5 Minuten gemessen. Die nachfolgende Tabelle 1 zeigt die erhaltenen Ergebnisse.

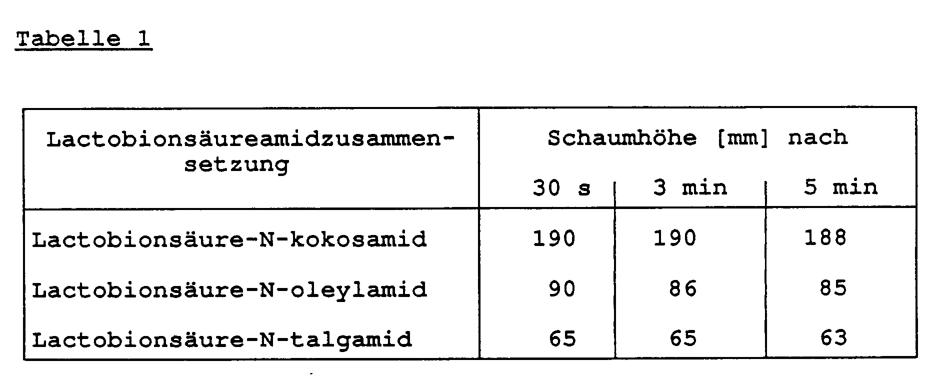

Die in Tabelle 1 dargestellten Ergebnisse belegen das gute Schaumvermögen der erfindungsgemäßen Lactobionsäureamidzusammensetzungen. Der erhaltene Schaum ist sehr feinporig und auch nach längerer Zeit noch stabil. Insbesondere mit Lactobionsäure-N-kokosamid läßt sich eine große für längere Zeit stabile Schaummenge erzeugen. Lactobionsäure-N-kokosamid ist daher sehr gut in kosmetischen Formulierungen, bei denen eine große und stabile Schaummenge erwünscht ist, beispielsweise in Schaumbädern oder Haarshampoos, einsetzbar.

### 2. b) Oberflächenspannung; kritische Micellenbildungskonzentration:

Für die erfindungsgemäßen Lactobionsäureamidzusammensetzungen wurde die Oberflächenspannung sowie die kritische Micellenbildungskonzentration (Critical Micellization concentration = C.M.C.) auf an sich bekannte Weise ermittelt. Hierfür wurde mit einem Tensiometer in Anlehnung an DIN 53914 die durch die Oberflächenspannung ausgeübte Kraft gemessen, die aufgebracht werden muß, um einen zur Flüssigkeitsoberfläche horizontal aufgehängten Ring aus der Flüssigkeitsoberfläche herauszuziehen. Tabelle 1a zeigt die erhaltenen Werte.

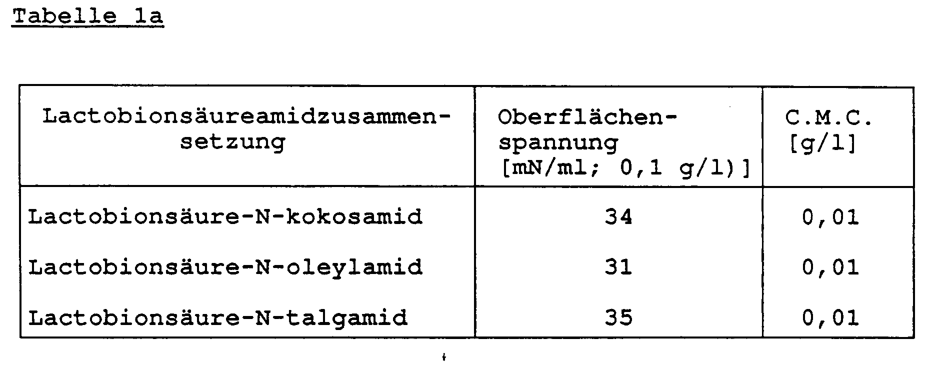

Die für die Oberflächenspannung und für die C.M.C. ermittelten Werte belegen die guten detergierenden Eigenschaften der erfindungsgemäßen Lactobionsäureamidzusammensetzungen für deren Verwendung als Tensidbestandteil in Wasch- und Reinigungszusammensetzungen.

### 3. Verwendung von Lactobionsäure-N-oleylamid als Emulgator:

Lactobionsäure-N-oleylamid, Wasser und Distelöl wurden in den in der nachfolgenden Tabelle 2 angegebenen Konzentrationen in einem Becherglas bei 60 bis 70 °C mit einem Homogenisator (Ultraturrax) homogenisiert. Zur Konservierung wurde den Emulsionen noch 0,1 Gew.-% Formaldehyd zugefügt. Die nachfolgende Tabelle 2 zeigt die erhaltenen Ergebnisse. Die Konzentrationen werden in Gew.-%, bezogen auf die Gesamtemulsion, angegeben.

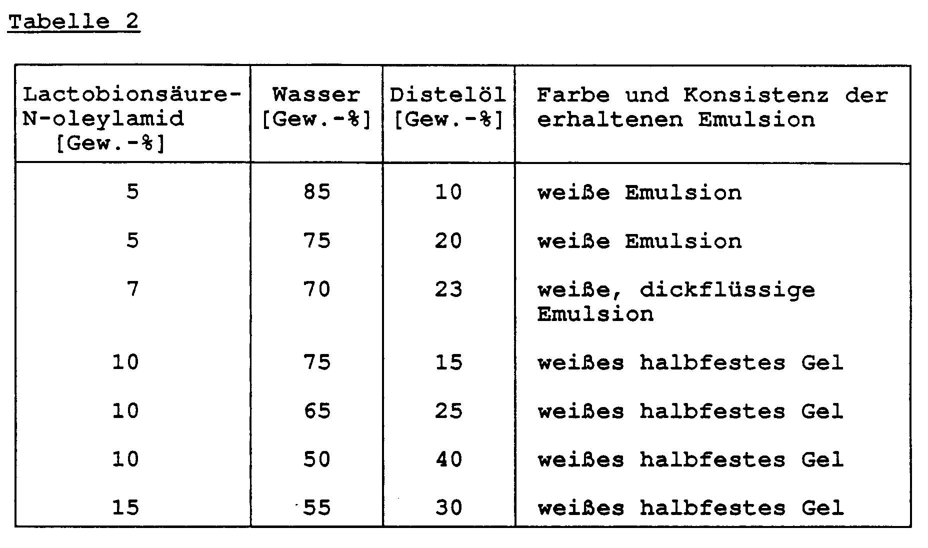

Ein weiterer Versuch wurde wie vorgehend beschrieben, jedoch unter Verwendung von Talgfett-11-ethoxylat als Ölphase durchgeführt. Dabei wurde folgendes Ergebnis erhalten:

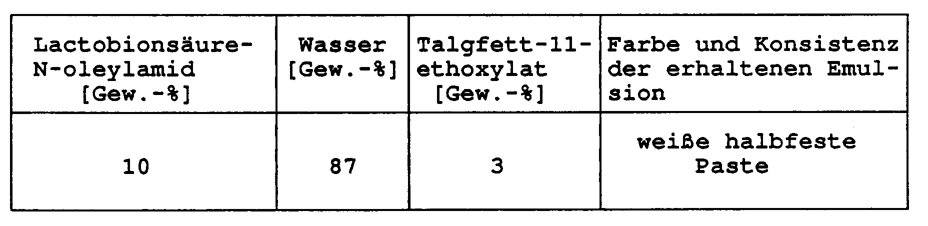

Auch nach 3 Wochen Lagerung konnten keine Änderungen in der Farbe und Konsistenz der erhaltenen Emulsionen festgestellt werden. Die mit Lactobionsäure-N-oleylamid hergestellten Emulsionen weisen also eine sehr gute Langzeitstabilität auf.

### 4. Verwendung von Lactobionsäure-N-oleylamid als Verdickungsmittel:

Es wurden wäßrige Lösungen mit 2 bis 12 % Lactobionsäure-N-oleylamid hergestellt, die bei 60 bis 70 °C mit einem Homogenisator homogenisiert wurden. Tabelle 3 zeigt die bei der Verwendung von Lactobionsäure-N-oleylamid als Verdickungsmittel erhaltenen Ergebnisse.

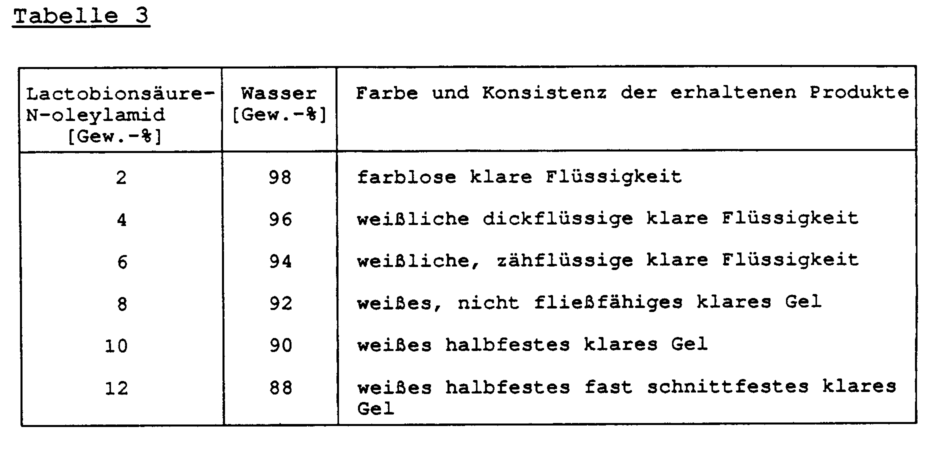

Die erhaltenen Ergebnisse zeigen die gute verdickende Wirkung von Lactobionsäure-N-oleylamid. Je nach eingesetzter Konzentration von Lactobionsäure-N-oleylamid können unterschiedlich starke Verdickungsgrade eingestellt werden. Die aufgrund der guten Löslichkeit von Lactobionsäure-N-oleylamid erzielbare Klarheit der Formulierungen ist dabei besonders vorteilhaft. Somit ist Lactobionsäure-N-oleylamid in idealer Weise als Verdickungsmittel, z.B. in kosmetischen Cremes, Salben, Pasten oder Gels, verwendbar.

### 5. Verwendung von Lactobionsäure-N-oleylamid oder Lactobionsäure-N-talgamid in Textilweichspülmitteln:

Mit Lactobionsäureamidzusammensetzungen wurden auch Weichspülversuche durchgeführt.

Für diese Versuche wurden Tücher à 10 x 10 cm aus Baumwollfrotteestoff geschnitten und unter Rühren in die zu testende wäßrige Lösung, die eine erfindungsgemäße Lactobionsäureamidzusammensetzung in einer Konzentration von 0,15 g/600 ml enthielt, vollständig eingetaucht. Anschließend wurden die Tücher von Hand ausgedrückt und an der Luft getrocknet. In analoger Weise wurde zum Vergleich ein Tuch nur mit Wasser behandelt. Die getrockneten Tücher wurden von 10 Personen auf ihre Weichheit geprüft, wobei Noten zwischen 1 und 4 vergeben werden konnten. Als Vergleich diente das nur mit Wasser behandelte Tuch, dessen Weichheit der Wert 0 zugeordnet wurde. Tabelle 4 zeigt die in der Beurteilung der Weichheit vergebenen Mittelwerte.

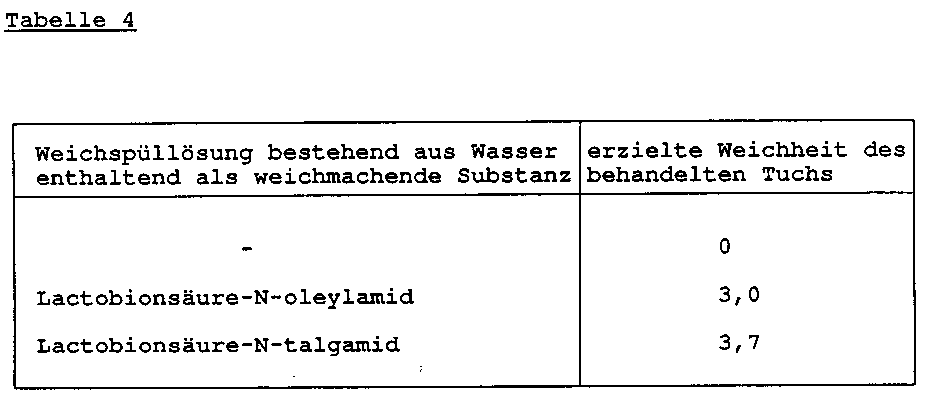

Auf analoge Weise wurden auch Versuche mit Lösungen durchgeführt, die Lactobionsäure-N-oleylamid bzw. Lactobionsäure-N-talgamid in einer Konzentration von 0,6 g/600 ml sowie 1,2 g/600 ml enthielten. Auch diese Werte entsprachen den in Tabelle 4 dargestellten Ergebnissen.

Die Ergebnisse zeigen, daß die verwendeten Lactobionsäureamidzusammensetzungen eine gute textilweichmachenden Wirkung besitzen.

## Patentansprüche

1. Lactobionsäureamidzusammensetzung aus Amiden der Lactobionsäure mit einem Gemisch primärer Fettamine, wobei das Fettamingemisch ein aus einem natürlich vorkommenden Fettsäuregemisch gewonnenes Fettamingemisch ist und mindestens 30 Gew.-% an Fettaminen mit einer Kettenlänge von 14 bis 18 Kohlenstoffatomen enthält.

2. Lactobionsäureamidzusammensetzung gemäß Anspruch 1, dadurch gekennzeichnet, daß das Fettamingemisch einen Anteil von mindestens 90 % an Fettaminen mit einer Kettenlänge von 16 bis 18 Kohlenstoffatomen enthält.

3. Lactobionsäureamidzusammensetzung gemäß Anspruch 2, dadurch gekennzeichnet, daß das Fettamingemisch einen Anteil von 5 bis 85 Gew.-% an einfach ungesättigten Fettaminen enthält.

4. Lactobionsäureamidzusammensetzung gemäß Anspruch 1, dadurch gekennzeichnet, daß das Fettamingemisch Kokosfettamin ist.

5. Lactobionsäurezusammensetzung gemäß Anspruch 1, dadurch gekennzeichnet, daß das Fettamingemisch Talgamin ist.

6. Lactobionsäureamidzusammensetzung gemäß Anspruch 1, dadurch gekennzeichnet, daß das Fettamingemisch hydriertes Talgamin ist.

7. Lactobionsäureamidzusammensetzung gemäß Anspruch 1, dadurch gekennzeichnet, daß das Fettamingemisch Oleylamin ist.

8. Verwendung von Lactobionsätireamidzusammensetzungen gemäß Anspruch 1 bis 7 als detergierend, emulgierend, schaumstabilisierend und/oder verdickend wirksame grenzflächenaktive Bestandteile in Wasch-, Spül- und/oder Reinigungszusammensetzungen oder kosmetischen Formulierungen.

9. Verwendung nach Anspruch 8, dadurch gekennzeichnet, daß Lactobionsäureamidzusammensetzungen gemäß Anspruch 1 bis 8 als weichmachende Bestandteile in Weichspülmitteln verwendet werden.

10. Verwendung nach Anspruch 8, dadurch gekennzeichnet, daß Lactobionsäureamidzusammensetzungen gemäß Anspruch 8 als Verdickungsmittel verwendet werden.

11. Verfahren zur Herstellung von Lactobionsäureamidzusammensetzungen gemäß Anspruch 1, dadurch gekennzeichnet, daß man Lactobionsäure oder ein reaktives Lactobionsäurederivat mit einem aus einem natürlich vorkommenden Fettsäuregemisch gewonnenem Fettamingemisch, das mindestens 30 Gew.-% an Fettaminen mit einer Kettenlänge von 14 bis 18 Kohlenstoffatomen enthält, umsetzt.

12. Verfahren nach Anspruch 11, dadurch gekennzeichnet, daß die Umsetzung in einem niederen Alkylalkohol durchgeführt wird.

## Claims

1. A lactobionic acid amide composition consisting of amides of lactobionic acid with a mixture of primary fatty amines, wherein the fatty amine mixture is a fatty amine mixture obtained from a naturally occurring fatty acid mixture and contains at least 30% by weight fatty amines with a chain length of 14 to 18 carbon atoms.

2. A lactobionic acid amide composition according to Claim 1, characterized in that the fatty amine mixture has a content of at least 90% fatty amines having a chain length of 16 to 18 carbon atoms.

3. A lactobionic acid amide composition according to Claim 2, characterised in that the fatty amine mixture has a content of 5 to 85% by weight of singly unsaturated fatty amines.

4. A lactobionic acid amide composition according to Claim 1, characterized in that the fatty amine mixture is coconut oil amine.

5. A lactobionic acid amide composition according to Claim 1, characterised in that the fatty amine mixture is tallow amine.

6. A lactobionic acid amide composition according to Claim 1, characterised in that the fatty amine mixture is hydrogenated tallow amine.

7. A lactobionic acid amide composition according to Claim 1, characterised in that the fatty amine mixture is oleylamine.

8. The use of lactobionic acid amide compositions according to Claims 1 to 7 as detergent, emulsifying, foam-stabilising and/or thickening surface-active constituents in detergent, rinsing and/or cleaning compositions or cosmetic formulations.

9. The use according to Claim 8, characterised in that lactobionic acid amide compositions according to Claims 1 to 7 are used as softening constituents in fabric-softening agents.

10. The use according to Claim 8, characterised in that lactobionic acid amide compositions according to Claim 7 are used as thickening agents.

11. A method for preparing lactobionic acid amide compositions according to Claim 1, characterised in that lactobionic acid or a reactive lactobionic acid derivative is reacted with a fatty amine mixture obtained from a naturally occurring fatty acid mixture, which fatty amine mixture contains at least 30% by weight fatty amines with a chain length of 14 to 18 carbon atoms.

12. A method according to Claim 11, characterised in that the reaction is performed in a lower alkyl alcohol.

## Revendications

1. Composition d'amide de l'acide lactobionique constituée d'amides de l'acide lactobionique avec un mélange d'amines grasses primaires, le mélange d'amines grasses étant un mélange d'amines grasses obtenu à partir d'un mélange d'acides gras que l'on trouve dans la nature et contenant au moins 30% en poids d'amines grasses ayant une longueur de chaîne de 14 à 18 atomes de carbone.

2. Composition d'amide de l'acide lactobionique selon la revendication 1, caractérisée en ce que le mélange d'amines grasses contient une proportion d'au moins 90% d'amines grasses ayant une longueur de chaîne de 16 à 18 atomes de carbone.

3. Composition d'amide de l'acide lactobionique selon la revendication 2, caractérisée en ce que le mélange d'amines grasses contient une proportion de 5 à 85% en poids d'amines grasses monoinsaturées.

4. Composition d'amide de l'acide lactobionique selon la revendication 1, caractérisée en ce que le mélange d'amines grasses est une amine grasse de coprah.

5. Composition d'acide lactobionique selon la revendication 1, caractérisée en ce que le mélange d'amines grasses est une antine de suif.

6. Composition d'amide de l'acide lactobionique selon la revendication 1, caractérisée en ce que le mélange d'amines grasses est une amine de suif hydrogénée.

7. Composition d'amide de l'acide lactobionique selon la revendication 1, caractérisée en ce que le mélange d'amines grasses est l'oléylamine.

8. Utilisation de compositions d'amide de l'acide lactobionique selon les revendications 1 à 7 comme constituants tensioactifs à action détergente, émulsifiante, stabilisatrice de mousse et/ou épaississante dans des compositions de lavage, de rinçage et/ou de nettoyage ou des formulations cosmétiques.

9. Utilisation selon la revendication 8, caractérisée en ce qu'on utilise des compositions d'amide d'acide lactobionique selon les revendications 1 à 8 comme constituants plastifiants dans des produits de rinçage doux.

10. Utilisation selon la revendication 8, caractérisée en ce qu'on utilise des compositions d'amides de l'acide lactobionique selon la revendication 8 comme agents épaississants.

11. Procédé de préparation de compositions d'amides de l'acide lactobionique selon la revendication 1, caractérisé en ce qu'on fait réagir de l'acide lactobionique ou un dérivé réactif d'acide lactobionique avec un mélange d'amines grasses obtenu à partir d'un mélange d'acides gras que l'on trouve dans la nature, et qui contient au moins 30% en poids d'amines grasses ayant une longueur de chaîne de 14 à 18 atomes de carbone.

12. Procédé selon la revendication 11, caractérisé en ce qu'on conduit la réaction dans un alcool d'alkyle inférieur.
